# EUROPÄISCHE PATENTANMELDUNG

(11) **EP 1 574 582 A1**
(43) Veröffentlichungstag der Anmeldung: **14.09.2005**
(21) Anmeldenummer: 05003969.2
(22) Anmeldetag: 24.02.2005
(51) Int. Cl.: C12P 13/08

(54) **Verfahren zur Herstellung von L-Aminosäuren unter Verwendung coryneformer Bakterien**

(30) Priorität: 09.03.2004 DE 102004011248
(71) Anmelder: Degussa AG, 40474 Düsseldorf (DE)
(72) Erfinder: Bathe, Brigitte, Dr., 33154 Salzkrotten (DE)

(57) **Zusammenfassung**

Die Erfindung betrifft ein Verfahren zur Herstellung von L-Aminosäuren, bei dem man folgende Schritte durchführt:
a) Fermentation der die gewünschte L-Aminosäure produzierenden coryneforme Bakterien, in denen man zumindest das für den Transkriptionsregulator TipA kodierende Gen abschwächt,
b) Anreicherung der gewünschten L-Aminosäure im Medium oder in den Zellen der Bakterien, und
c) Isolierung der L-Aminosäure,
und gegebenenfalls Bakterien einsetzt, in denen man zusätzlich weitere Gene des Biosyntheseweges der gewünschten L-Aminosäure verstärkt, oder Bakterien einsetzt, in denen die Stoffwechselwege zumindest teilweise ausgeschaltet sind, die die Bildung der gewünschten L-Aminosäure verringern.

## Beschreibung

Gegenstand der Erfindung ist ein Verfahren zur Herstellung von L-Aminosäuren, insbesondere L-Lysin, unter Verwendung coryneformer Bakterien, in denen das tipA-Gen kodierend für den Transkriptionsregulator TipA abgeschwächt ist.

### Stand der Technik

L-Aminosäuren, insbesondere L-Lysin, finden in der Humanmedizin und in der pharmazeutischen Industrie, in der Lebensmittelindustrie und ganz besonders in der Tierernährung Anwendung.

Es ist bekannt, dass Aminosäuren durch Fermentation von Stämmen coryneformer Bakterien, insbesondere Corynebacterium glutamicum hergestellt werden. Wegen der großen Bedeutung wird ständig an der Verbesserung der Herstellverfahren gearbeitet. Verfahrensverbesserungen können fermentationstechnische Maßnahmen wie zum Beispiel Rührung und Versorgung mit Sauerstoff, oder die Zusammensetzung der Nährmedien wie zum Beispiel die Zuckerkonzentration während der Fermentation, oder die Aufarbeitung zur Produktform durch zum Beispiel Ionenaustauschchromatographie oder die intrinsischen Leistungseigenschaften des Mikroorganismus selbst betreffen.

Zur Verbesserung der Leistungseigenschaften dieser Mikroorganismen werden Methoden der Mutagenese, Selektion und Mutantenauswahl angewendet. Auf diese Weise erhält man Stämme, die resistent gegen Antimetabolite wie z.B. Lysin-Analogon S-(2-Aminoethyl)-Cystein oder auxotroph für regulatorisch bedeutsame Metabolite sind und L-Aminosäuren produzieren.

Seit einigen Jahren werden ebenfalls Methoden der rekombinanten DNA-Technik zur Stammverbesserung L-Aminosäure produzierender Stämme von Corynebacterium glutamicum eingesetzt, indem man einzelne Aminosäure-Biosynthesegene amplifiziert und die Auswirkung auf die L-Aminosäure-Produktion untersucht.

### Aufgabe der Erfindung

Die Erfinder haben sich die Aufgabe gestellt, neue Grundlagen für verbesserte Verfahren zur fermentativen Herstellung von L-Aminosäuren, insbesondere L-Lysin, mit coryneformen Bakterien bereitzustellen.

### Beschreibung der Erfindung

Gegenstand der Erfindung ist ein Verfahren zur Herstellung von L-Aminosäuren, unter Verwendung von coryneformen Bakterien, in denen man zumindest die für den Transkriptionsregulator TipA kodierende Nukleotidsequenz abschwächt, insbesondere ausschaltet oder auf niedrigem Niveau exprimiert.

Gegenstand dieser Erfindung ist weiterhin ein Verfahren zur Herstellung von L-Aminosäuren, in dem folgende Schritte durchführt werden:
a) Fermentation der L-Aminosäure produzierenden coryneformen Bakterien, in denen zumindest die für den Transkriptionsregulator TipA kodierende Nukleotidsequenz abgeschwächt, insbesondere ausgeschaltet oder auf niedrigem Niveau exprimiert wird.
b) Anreicherung der L-Aminosäuren im Medium oder in den Zellen der Bakterien; und
c) Isolierung der gewünschten L-Aminosäuren, wobei gegebenenfalls Bestandteile der Fermentationsbrühe und/oder der Biomasse in Anteilen oder in ihren Gesamtmengen im Endprodukt verbleiben.

Die eingesetzten coryneformen Bakterien produzieren bevorzugt bereits vor der Abschwächung des für den Transkriptionsregulator TipA kodierenden tipA-Gens L-Aminosäuren, insbesondere L-Lysin.

Es wurde gefunden, dass coryneforme Bakterien nach Abschwächung des für den Transkriptionsregulator TipA kodierenden tipA-Gens in verbesserter Weise L-Aminosäuren, insbesondere L-Lysin produzieren.

Transkriptionsregulatoren sind solche Proteine, die durch eine spezifische Proteinstruktur, genannt Helix-Turn-Helix-Motiv, an DNA binden können und so die Transkription anderer Gene entweder verstärken oder abschwächen können. Es wurde gefunden, dass die Funktion des Transkriptionsregulators TipA aus Corynebacterium glutamicum die Repression von Genen, die an L-Aminosäurebiosynthesen, insbesondere an der L-Lysinbiosynthese beteiligt sind, bewirkt. Die Abschwächung des Transkriptionsregulators TipA verbessert die L-Lysinproduktion in den entsprechenden coryneformen Bakterien.

Die Nukleotidsequenz des für den Transkriptionsregulator TipA von Corynebacterium glutamicum kodierenden Gens kann der Patentanmeldung EP1108790 als Sequenz Nr. 2871 sowie als Sequenz Nr. 7068 entnommen werden.

Die Nukleotidsequenz ist ebenfalls in der Datenbank des National Center for Biotechnology Information (NCBI) der National Library of Medicin (Bethesda, MD, USA) unter der Accession Number AX122955 und unter der Accession Number AX127152 hinterlegt.

Die in der angegebenen Textstelle beschriebene Sequenz kodierend für den Transkriptionsregulator TipA kann erfindungsgemäß verwendet werden. Weiterhin können Allele des Transkriptionsregulators TipA verwendet werden, die sich aus der Degeneriertheit des genetischen Kodes oder durch funktionsneutrale Sinnmutationen ("sense mutations") ergeben.

Werden im folgenden L-Aminosäuren oder Aminosäuren erwähnt, sind damit eine oder mehrere Aminosäuren einschließlich ihrer Salze, ausgewählt aus der Gruppe L-Asparagin, L-Threonin, L-Serin, L-Glutamat, L-Glycin, L-Alanin, L-Cystein, L-Valin, L-Methionin, L-Isoleucin, L-Leucin, L-Tyrosin, L-Phenylalanin, L-Histidin, L-Lysin, L-Tryptophan und L-Arginin gemeint. Besonders bevorzugt ist L-Lysin.

Wenn im Folgenden L-Lysin oder Lysin erwähnt werden, sind damit nicht nur die Basen, sondern sind auch die Salze wie z.B. Lysin-Monohydrochlorid oder Lysin-Sulfat gemeint.

Bevorzugte Ausführungsformen finden sich in den Ansprüchen.

Der Begriff "Abschwächung" beschreibt in diesem Zusammenhang die Verringerung oder Ausschaltung der intrazellulären Aktivität eines oder mehrerer Enzyme (Proteine) in einem Mikroorganismus, die durch die entsprechende DNA kodiert werden, indem man beispielsweise einen schwachen Promotor verwendet oder ein Gen bzw. Allel verwendet, das für ein entsprechendes Enzym mit einer niedrigen Aktivität kodiert bzw. das entsprechende Gen oder Enzym (Protein) inaktiviert und gegebenenfalls diese Maßnahmen kombiniert.

Durch die Maßnahmen der Abschwächung wird die Aktivität oder Konzentration des entsprechenden Proteins im allgemeinen auf 0 bis 75%, 0 bis 50%, 0 bis 25%, 0 bis 10% oder 0 bis 5% der Aktivität oder Konzentration des Wildtyp-Proteins, beziehungsweise der Aktivität oder Konzentration des Proteins im Ausgangs-Mikroorganismus, herabgesenkt.

Die Herabsetzung der Proteinkonzentration ist über 1- und 2-dimensionale Proteingelauftrennung und anschließende optische Identifizierung der Proteinkonzentration mit entsprechender Auswertesoftware im Gel nachweisbar. Eine gebräuchliche Methode zur Präparation der Proteingele bei coryneformen Bakterien und zur Identifizierung der Proteine ist die von Hermann et al. (Electrophoresis, 22:1712-23 (2001)) beschriebene Vorgehensweise.

Die Proteinkonzentration kann ebenfalls durch Western-Blot-Hybridisierung mit einem für das nachzuweisende Protein spezifischen Antikörper (Sambrook et al., Molecular Cloning: A Laboratory Manual, 2^{nd} Ed., Cold Spring Harbor Laboratory Press, Cold Spring Harbor, N.Y., 1989) und anschließender optischer Auswertung mit entsprechender Software zur Konzentrationsbestimmung (Lohaus und Meyer (1998) Biospektrum 5:32-39; Lottspeich (1999) Angewandte Chemie 111:2630-2647) analysiert werden. Die Aktivität von DNA-bindenden Proteinen kann mittels DNA Band-Shift-Assays (auch als Gelretardation bezeichnet) gemessen werden (Wilson et al. (2001) Journal of Bacteriology 183:2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: A Laboratory Manual, 2^{nd} Ed., Cold Spring Harbor Laboratory Press, Cold Spring Harbor, N.Y., 1989).

Die Mikroorganismen, die Gegenstand der vorliegenden Erfindung sind, können Aminosäuren aus Glucose, Saccharose, Laktose, Fructose, Maltose, Melasse, Stärke, Cellulose oder aus Glycerin und Ethanol herstellen. Es kann sich um Vertreter coryneformer Bakterien insbesondere der Gattung Corynebacterium handeln. Bei der Gattung Corynebacterium ist insbesondere die Art Corynebacterium glutamicum zu nennen, die in der Fachwelt für ihre Fähigkeit bekannt ist, L-Aminosäuren zu produzieren.

Geeignete Stämme der Gattung Corynebacterium, insbesondere der Art Corynebacterium glutamicum, sind besonders die bekannten Wildtypstämme
Corynebacterium glutamicum ATCC13032
Corynebacterium acetoglutamicum ATCC15806
Corynebacterium acetoacidophilum ATCC13870
Corynebacterium melassecola ATCC17965
Corynebacterium thermoaminogenes FERM BP-1539
Brevibacterium flavum ATCC14067
Brevibacterium lactofermentum ATCC13869 und
Brevibacterium divaricatum ATCC14020
und daraus hergestellte L-Aminosäuren produzierende Mutanten bzw. Stämme wie beispielsweise die L-Lysin produzierenden Stämme
Corynebacterium glutamicum FERM-P 1709
Brevibacterium flavum FERM-P 1708
Brevibacterium lactofermentum FERM-P 1712
Corynebacterium glutamicum FERM-P 6463
Corynebacterium glutamicum FERM-P 6464 und
Corynebacterium glutamicum DSM 5715.

Zur Erzielung einer Abschwächung können entweder die Expression des für den Transkriptionsregulator TipA kodierenden Gens oder die regulatorischen Eigenschaften der Genprodukte herabgesetzt oder ausgeschaltet werden. Gegebenenfalls werden beide Maßnahmen kombiniert.

Die Genexpression kann durch geeignete Kulturführung oder durch genetische Veränderung (Mutation) der Signalstrukturen der Genexpression verringert werden. Signalstrukturen der Genexpression sind beispielsweise Repressorgene, Aktivatorgene, Operatoren, Promotoren, Attenuatoren, Ribosomenbindungsstellen, das Startkodon und Terminatoren. Angaben hierzu findet der Fachmann z.B. in der Patentanmeldung WO 96/15246, bei Boyd und Murphy (Journal of Bacteriology 170: 5949 (1988)), bei Voskuil und Chambliss (Nucleic Acids Research 26: 3548 (1998), bei Jensen und Hammer (Biotechnology and Bioengineering 58: 191 (1998)), bei Pätek et al. (Microbiology 142: 1297 (1996)) und in bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995) oder dem von Winnacker ("Gene und Klone", VCH Verlagsgesellschaft, Weinheim, Deutschland, 1990).

Eine weitere Methode zur spezifischen Verringerung der Genexpression ist die Antisense-Technik, wobei kurze Oligodesoxyukleotide oder Vektoren zur Synthese längerer antisense-RNA in die Zielzellen gebracht werden. Die Antisense-RNA kann dort an komplementäre Abschnitte spezifischer mRNAs binden und deren Stabilität verringern oder die Translatierbarkeit blocken. Ein Beispiel hierzu findet der Fachmann bei Srivastava et al. (Applied Environmental Microbiology 2000 Oct; 66 (10): 4366 - 4371).

Mutationen, die zu einer Veränderung bzw. Herabsetzung der katalytischen Eigenschaften von Enzymproteinen führen, sind aus dem Stand der Technik bekannt; als Beispiele seien die Arbeiten von Qiu und Goodman (Journal of Biological Chemistry 272: 8611-8617 (1997)), Sugimoto et al. (Bioscience Biotechnology and Biochemistry 61: 1760-1762 (1997)) und Möckel ("Die Threonindehydratase aus Corynebacterium glutamicum: Aufhebung der allosterischen Regulation und Struktur des Enzyms", Berichte des Forschungszentrums Jülichs,Jül-2906, ISSN09442952, Jülich, Deutschland, 1994) genannt. Zusammenfassende Darstellungen können bekannten Lehrbüchern der Genetik und Molekularbiologie wie z.B. dem von Hagemann ("Allgemeine Genetik", Gustav Fischer Verlag, Stuttgart, 1986) entnommen werden.

Als Mutationen kommen Transitionen, Transversionen, Insertionen und Deletionen in Betracht. In Abhängigkeit von der Wirkung des Aminosäureaustausches auf die Enzymaktivität wird von Fehlsinnmutationen ("missense mutations") oder Nichtsinnmutationen ("nonsense mutations") gesprochen. Insertionen oder Deletionen von mindestens einem Basenpaar in einem Gen führen zu Rasterverschiebungsmutationen ("frame shift mutations"), in deren Folge falsche Aminosäuren eingebaut werden oder die Translation vorzeitig abbricht. Deletionen von mehreren Kodonen führen typischerweise zu einem vollständigen Ausfall der Enzymaktivität. Anleitungen zur Erzeugung derartiger Mutationen gehören zum Stand der Technik und können bekannten Lehrbüchern der Genetik und Molekularbiologie wie z.B. dem Lehrbuch von Knippers ("Molekulare Genetik", 6. Auflage, Georg Thieme Verlag, Stuttgart, Deutschland, 1995), dem von Winnacker ("Gene und Klone", VCH Verlagsgesellschaft, Weinheim, Deutschland, 1990) oder dem von Hagemann ("Allgemeine Genetik", Gustav Fischer Verlag, Stuttgart, 1986) entnommen werden.

Eine gebräuchliche Methode, Gene von C. glutamicum zu mutieren, ist die von Schwarzer und Pühler (Bio/Technology 9, 84-87 (1991)) beschriebene Methode der Gen-Unterbrechung ("gene disruption") und des Gen-Austauschs ("gene replacement").

Bei der Methode der Gen-Unterbrechung wird ein zentraler Teil der Kodierregion des interessierenden Gens in einen Plasmidvektor kloniert, der in einem Wirt (typischerweise E. coli), nicht aber in C. glutamicum replizieren kann. Als Vektoren kommen beispielsweise pSUP301 (Simon et al., Bio/Technology 1, 784-791 (1983)), pK18mob oder pK19mob (Schäfer et al., Gene 145, 69-73 (1994)), pK18mobsacB oder pK19mobsacB (Jäger et al., Journal of Bacteriology 174: 5462-65 (1992)), pGEM-T (Promega corporation, Madison, WI, USA), pCR2.1-TOPO (Shuman (1994). Journal of Biological Chemistry 269:32678-84; US-Patent 5,487,993), pCR®Blunt (Firma Invitrogen, Groningen, Niederlande; Bernard et al., Journal of Molecular Biology, 234: 534-541 (1993)) oder pEM1 (Schrumpf et al, 1991, Journal of Bacteriology 173:4510-4516) in Frage. Der Plasmidvektor, der das zentrale Teil der Kodierregion des Gens enthält, wird anschließend durch Konjugation oder Transformation in den gewünschten Stamm von C. glutamicum überführt. Die Methode der Konjugation ist beispielsweise bei Schäfer et al. (Applied and Environmental Microbiology 60, 756-759 (1994)) beschrieben. Methoden zur Transformation sind beispielsweise bei Thierbach et al. (Applied Microbiology and Biotechnology 29, 356-362 (1988)), Dunican und Shivnan (Bio/Technology 7, 1067-1070 (1989)) und Tauch et al. (FEMS Microbiological Letters 123, 343-347 (1994)) beschrieben. Nach homologer Rekombination mittels eines "cross-over"-Ereignisses wird die Kodierregion des betreffenden Gens durch die Vektorsequenz unterbrochen und man erhält zwei unvollständige Allele, denen jeweils das 3'- bzw. das 5'-Ende fehlt. Diese Methode wurde beispielsweise von Fitzpatrick et al. (Applied Microbiology and Biotechnology 42, 575-580 (1994)) zur Ausschaltung des recA-Gens von C. glutamicum verwendet.

Bei der Methode des Genaustausches ("gene replacement") wird eine Mutation wie z.B. eine Deletion, Insertion oder Basenaustausch in dem interessierenden Gen in-vitro hergestellt. Das hergestellte Allel wird wiederum in einen für C. glutamicum nicht replikativen Vektor kloniert und dieser anschließend durch Transformation oder Konjugation in den gewünschten Wirt von C. glutamicum überführt. Nach homologer Rekombination mittels eines ersten, Integration bewirkenden "cross-over"-Ereignisses und eines geeigneten zweiten, eine Exzision bewirkenden "cross-over"-Ereignisses im Zielgen bzw. in der Zielsequenz erreicht man den Einbau der Mutation bzw. des Allels. Diese Methode wurde beispielsweise von Peters-Wendisch et al. (Microbiology 144, 915 - 927 (1998)) verwendet, um das pyc-Gen von C. glutamicum durch eine Deletion auszuschalten.

In das für den Transkriptionsregulator TipA kodierende Gen kann auf diese Weise eine Deletion, Insertion oder ein Basenaustausch eingebaut werden.

Zusätzlich kann es für die Produktion von L-Aminosäuren vorteilhaft sein, zusätzlich zur Abschwächung des für den Transkriptionsregulator TipA kodierenden Gens eines oder mehrere Enzyme des jeweiligen Biosyntheseweges, der Glykolyse, der Anaplerotik, des Zitronensäure-Zyklus, des Pentosephosphat-Zyklus, des Aminosäure-Exports und gegebenenfalls regulatorische Proteine zu verstärken, insbesondere überzuexprimieren.

Der Begriff "Verstärkung" bzw. "Verstärken" beschreibt in diesem Zusammenhang die Erhöhung der intrazellulären Aktivität eines oder mehrerer Enzyme bzw. Proteine in einem Mikroorganismus, die durch die entsprechende DNA kodiert werden, indem man beispielsweise die Kopienzahl des Gens bzw. der Gene erhöht, einen starken Promotor oder ein Gen verwendet, das für ein entsprechendes Enzym bzw. Protein mit einer hohen Aktivität kodiert und gegebenenfalls diese Maßnahmen kombiniert.

Durch die Maßnahmen der Verstärkung, insbesondere Überexpression, wird die Aktivität oder Konzentration des entsprechenden Proteins im allgemeinen um mindestens 10%, 25%, 50%, 75%, 100%, 150%, 200%, 300%, 400% oder 500%, maximal bis 1000% oder 2000% bezogen auf die des Wildtyp-Proteins beziehungsweise der Aktivität oder Konzentration des Proteins im Ausgangs-Mikroorganismus erhöht.

Die Verwendung endogener Gene wird im allgemeinen bevorzugt. Unter "endogenen Genen" oder "endogenen Nukleotidsequenzen" versteht man die in der Population einer Art vorhandenen Gene beziehungsweise Nukleotidsequenzen.

So kann für die Herstellung von L-Lysin neben der Abschwächung des für den Transkriptionsregulator TipA kodierenden Gens eines oder mehrere der Gene ausgewählt aus der Gruppe
- das für eine feed-back resistente Aspartatkinase kodierende Gen lysC (Accession No.P26512, EP-B-0387527; EP-A-0699759; WO 00/63388),
- gleichzeitig das für das Lysin-Export-Protein kodierende Gen lysE (DE-A-195 48 222),
- das für die Glyceraldehyd-3-Phosphat Dehydrogenase kodierende Gen gap (Eikmanns (1992). Journal of Bacteriology 174:6076-6086),
- das für die Pyruvat Carboxylase kodierende Gen pyc (EP-A-1083225),
- das für die Glucose-6-Phosphat Dehydrogenase kodierende Gen zwf (JP-A-09224661, WO 01/70995),
- das für die Malat:Chinon Oxidoreduktase kodierende Gen mqo (Molenaar et al., European Journal of Biochemistry 254, 395-403 (1998); EP-A-1038969),
- das für das Zwa1-Protein kodierende Gen zwa1 (DE: 19959328.0, DSM 13115; EP-A-1111062),
- das für die Triosephosphat Isomerase kodierende Gen tpi (Eikmanns (1992), Journal of Bacteriology 174:6076-6086),
- das für die 3-Phosphoglycerat Kinase kodierende Gen pgk (Eikmanns (1992), Journal of Bacteriology 174:6076-6086),
- das für die Dihydrodipicolinat-Synthase kodierende Gen dapA (EP-B 0 197 335),
verstärkt, insbesondere überexprimiert werden.

Weiterhin kann es für die Produktion von Aminosäuren, insbesondere L-Lysin, vorteilhaft sein, zusätzlich zur Abschwächung des für den Transkriptionsregulator TipA kodierenden Gens gleichzeitig eines oder mehrere der Gene, ausgewählt aus der Gruppe
- das für ein Katabolit-Kontroll-Protein A kodierende Gen ccpA1 (EP1311685),
- das für die Phosphoenolpyruvat-Carboxykinase kodierende Gen pck (DSM 13047, EP-A-1094111),
- das für die Glucose-6-Phosphat-Isomerase kodierende Gen pgi (DSM 12969, EP-A-1087015, WO 01/07626),
- das für die Pyruvat-Oxidase kodierende Gen poxB (DSM 13114, EP-A-1096013),
- das für die Fruktose-Bisphosphat Aldolase kodierende Gen fda (Mol. Microbiol. 3 (11), 1625-1637 (1989); ACCESSION Number X17313),
- das für das Zwa2-Protein kodierende Gen zwa2 (DSM 13113, EP-A-1106693),
abzuschwächen, insbesondere die Expression zu verringern.

Schließlich kann es für die Produktion von Aminosäuren, vorteilhaft sein, neben der Abschwächung des für den Transkriptionsregulator TipA kodierenden Gens unerwünschte Nebenreaktionen auszuschalten (Nakayama: "Breeding of Amino Acid Producing Micro-organisms", in: Overproduction of Microbial Products, Krumphanzl, Sikyta, Vanek (eds.), Academic Press, London, UK, 1982).

Die erfindungsgemäß hergestellten Mikroorganismen sind ebenfalls Gegenstand der Erfindung und können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Produktion von L-Aminosäuren kultiviert werden. Eine Zusammenfassung über bekannte Kultivierungsmethoden ist im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D.C., USA, 1981) enthalten.

Als Kohlenstoffquelle können Zucker und Kohlehydrate wie z.B. Glucose, Saccharose, Laktose, Fructose, Maltose, Melasse, Stärke und Cellulose, Öle und Fette wie z.B. Sojaöl, Sonnenblumenöl, Erdnußöl und Kokosfett, Fettsäuren wie z.B. Palmitinsäure, Stearinsäure und Linolsäure, Alkohole wie z.B. Glycerin und Ethanol und organische Säuren wie z.B. Essigsäure verwendet werden. Diese Stoffe können einzeln oder als Mischung verwendet werden.

Als Stickstoffquelle können organische Stickstoff-haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium haltigen Salze verwendet werden. Das Kulturmedium muss weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe wie z.B. Antibiotika hinzugefügt werden. Um aerobe Bedingungen aufrechtzuerhalten, werden Sauerstoff oder Sauerstoffhaltige Gasmischungen wie z.B. Luft in die Kultur eingetragen. Die Temperatur der Kultur liegt normalerweise bei 20°C bis 45°C und vorzugsweise bei 25°C bis 40°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Methoden zur Bestimmung von L-Aminosäuren sind aus dem Stand der Technik bekannt. Die Analyse kann so wie bei Spackman et al. (Analytical Chemistry, 30, (1958), 1190) beschrieben durch Anionenaustauschchromatographie mit anschließender Ninhydrin Derivatisierung erfolgen, oder sie kann durch reversed phase HPLC erfolgen, so wie bei Lindroth et al. (Analytical Chemistry (1979) 51: 1167-1174) beschrieben.

Folgender Mikroorganismus wird als Reinkultur am 15. Februar 2002 bei der Deutschen Sammlung für Mikroorganismen und Zellkulturen (DSMZ, Braunschweig, Deutschland) gemäß Budapester Vertrag hinterlegt:
- Escherichia coli Top10/pCR2.1tipAint als DSM 14816.

Die vorliegende Erfindung wird im Folgenden anhand von Ausführungsbeispielen näher erläutert.

### Beispiel 1

### Herstellung eines Integrationsvektors für die Integrationsmutagenese des Gens tipA

Aus dem Stamm ATCC 13032 wird nach der Methode von Eikmanns et al. (Microbiology 140: 1817 - 1828 (1994)) chromosomale DNA isoliert.

Aufgrund der aus für C. glutamicum bekannten Sequenz des Gens tipA werden die folgenden Oligonukleotide für die Polymerase Kettenreaktion ausgewählt:

tipA-int1:
5` CGC CTT TAC ACA GAA GAC G 3`

tipA-int2:
5` GTG TAC CAC TGA CCG ATG C 3`

Die dargestellten Primer werden von der Firma MWG Biotech (Ebersberg, Deutschland) synthetisiert und nach der Standard-PCR-Methode von Innis et al. (PCR Protocols. A Guide to Methods and Applications, 1990, Academic Press) mit der Taq-Polymerase der Firma Boehringer Mannheim (Deutschland, Produktbeschreibung Taq DNA Polymerase, Product No. 1 146 165) die PCR Reaktion durchgeführt. Mit Hilfe der Polymerase-Kettenreaktion ermöglichen die Primer die Amplifikation eines 482 bp großen internen Fragmentes des tipA-Gens. Das so amplifizierte Produkt wird in einem 0,8%igen Agarosegel elektrophoretisch geprüft.

Das amplifizierte DNA-Fragment wird mit dem TOPO TA Cloning Kit der Firma Invitrogen Corporation (Carlsbad, CA, USA; Katalog Nummer K4500-01) in den Vektor pCR2.1-TOPO (Mead at al. (1991) Bio/Technology 9:657-663) ligiert.

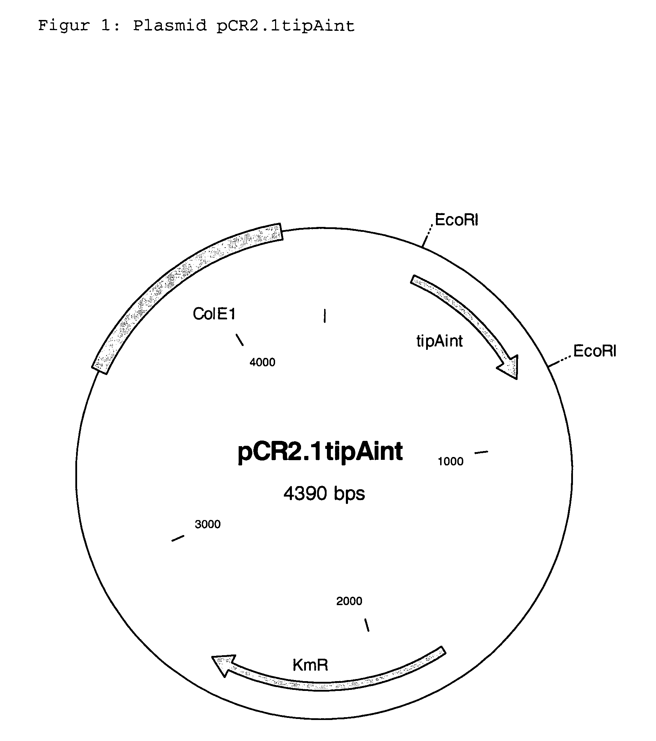

Anschließend wird der E. coli Stamm TOP10 mit dem Ligationsansatz (Hanahan, In: DNA cloning. A practical approach. Vol.I. IRL-Press, Oxford, Washington DC, USA, 1985) elektroporiert. Die Selektion von Plasmid-tragenden Zellen erfolgt durch Ausplattieren des Transformationsansatzes auf LB Agar (Sambrook et al., Molecular Cloning: A Laboratory Manual, 2^{nd} Ed., Cold Spring Harbor Laboratory Press, Cold Spring Harbor, N.Y., 1989), der mit 50 mg/l Kanamycin supplementiert worden ist. Plasmid-DNA wird aus einer Transformante mit Hilfe des QIAprep Spin Miniprep Kit der Firma Qiagen isoliert und durch Restriktion mit dem Restriktionsenzym EcoRI und anschließender Agarosegel-Elektrophorese (0,8%) überprüft. Das Plasmid wird pCR2.1tipAint genannt und ist in Figur 1 dargestellt.

Folgender Mikroorganismus wird als Reinkultur am 15. Februar 2002 bei der Deutschen Sammlung für Mikroorganismen und Zellkulturen (DSMZ, Braunschweig, Deutschland) gemäß Budapester Vertrag hinterlegt:
- Escherichia coli Top10/pCR2.1tipAint als DSM 14816.

### Beispiel 2

### Integrationsmutagenese des tipA-Gens in dem Stamm DSM 5715

Der in Beispiel 1 genannte Vektor pCR2.1tipAint wird nach der Elektroporationsmethode von Tauch et al.(FEMS Microbiological Letters, 123:343-347 (1994)) in Corynebacterium glutamicum DSM 5715 elektroporiert. Bei dem Stamm DSM 5715 handelt es sich um einen AEC resistenten Lysin-Produzenten, der Stamm ist in der EP-B-0435132 beschrieben. Der Vektor pCR2.1tipAint kann in DSM5715 nicht selbständig replizieren und bleibt nur dann in der Zelle erhalten, wenn er ins Chromosom von DSM 5715 integriert hat. Die Selektion von Klonen mit ins Chromosom integriertem pCR2.1tipAint erfolgt durch Ausplattieren des Elektroporationsansatzes auf LB Agar (Sambrook et al., Molecular Cloning: A Laboratory Manual, 2^{nd} Ed., Cold Spring Harbor Laboratory Press, Cold Spring Harbor, N.Y.), der mit 15 mg/l Kanamycin supplementiert worden ist.

Ein ausgewählter Kanamycin-resistenter Klon, der das in Beispiel 1 genannte Plasmid pCR2.1tipAint innerhalb des chromosomalen tipA-Gens von DSM5715 inseriert hat, wurde als DSM5715::pCR2.1tipAint bezeichnet.

### Beispiel 3

### Herstellung von Lysin

Der in Beispiel 2 erhaltene C. glutamicum Stamm DSM5715::pCR2.1tipAint wird in einem zur Produktion von Lysin geeigneten Nährmedium kultiviert und der Lysingehalt im Kulturüberstand bestimmt.

Dazu wird der Stamm zunächst auf Agarplatte mit dem entsprechenden Antibiotikum (Hirn-Herz Agar mit Kanamycin (25 mg/l) für 24 Stunden bei 33°C inkubiert. Ausgehend von dieser Agarplattenkultur wird eine Vorkultur angeimpft (10 ml Medium im 100 ml Erlenmeyerkolben). Als Medium für die Vorkultur wird das Vollmedium CgIII verwendet.

| Medium Cg III | |
|---|---|
| NaCl | 2,5 g/l |
| Bacto-Pepton | 10 g/l |
| Bacto-Yeast-Extrakt | 10 g/l |
| Glucose (getrennt autoklaviert) | 2% (w/v) |

Der pH-Wert wird auf pH 7.4
eingestellt

Diesem wird Kanamycin (25 mg/l) zugesetzt. Die Vorkultur wird 16 Stunden bei 33°C bei 240 rpm auf dem Schüttler inkubiert. Von dieser Vorkultur wird eine Hauptkultur angeimpft, so dass die Anfangs-OD (660 nm) der Hauptkultur 0,1 OD beträgt. Für die Hauptkultur wird das Medium MM verwendet.

| Medium MM | |
|---|---|
| CSL (Corn Steep Liquor) | 5 g/l |
| MOPS (Morpholinopropansulfonsäure) | 20 g/l |
| Glucose (getrennt autoklaviert) | 50g/l |

| Salze: | |
|---|---|
| (NH₄)₂SO₄) | 25 g/l |
| KH₂PO₄ | 0,1 g/l |
| MgSO₄ * 7 H₂O | 1,0 g/l |
| CaCl₂ * 2 H₂O | 10 mg/l |
| FeSO₄ * 7 H₂O | 10 mg/l |
| MnSO₄ * H₂O | 5,0mg/l |
| Biotin (sterilfiltriert) | 0,3 mg/l |
| Thiamin * HCl (sterilfiltriert) | 0,2 mg/l |
| Leucin (sterilfiltriert) | 0,1 g/l |
| CaCO₃ | 25 g/l |

CSL, MOPS und die Salzlösung werden mit Ammoniakwasser auf pH 7 eingestellt und autoklaviert. Anschließend werden die sterilen Substrat- und Vitaminlösungen zugesetzt, sowie das trocken autoklavierte CaCO₃ zugesetzt.

Die Kultivierung erfolgt in 10 ml Volumen in 100 ml Erlenmeyerkolben mit Schikanen. Es wird Kanamycin (25 mg/l) zugesetzt. Die Kultivierung erfolgt bei 33°C und 80% Luftfeuchtigkeit.

Nach 72 Stunden wird die OD bei einer Meßwellenlänge von 660 nm mit dem Biomek 1000 (Beckmann Instruments GmbH, München) ermittelt. Die gebildete Lysinmenge wird mit einem Aminosäureanalysator der Firma Eppendorf-BioTronik (Hamburg, Deutschland) durch Ionenaustauschchromatographie und Nachsäulenderivatisierung mit Ninhydrindetektion bestimmt .

In Tabelle 1 ist das Ergebnis des Versuchs dargestellt.

**Tabelle 1**

| Stamm | OD (660 nm) | Lysin-HCl g/l |
|---|---|---|
| DSM5715 | 8,2 | 13,6 |
| DSM5715::pCR2.1tipAint | 10,5 | 15,1 |

### Kurze Beschreibung der Figur:

Figur 1: Karte des Plasmids pCR2.1tipAint.

Bei der Angabe der Basenpaarzahlen handelt es sich um Näherungswerte, die im Rahmen der Reproduzierbarkeit von Messungen erhalten werden.

Die verwendeten Abkürzungen und Bezeichnungen haben folgende Bedeutung:
- KmR:: Kanamycin Resistenz-Gen
- EcoRI:: Schnittstelle des Restriktionsenzyms EcoRI
- tipAint:: internes Fragment des tipA-Gens
- ColE1:: Replikationsursprung des Plasmides ColE1

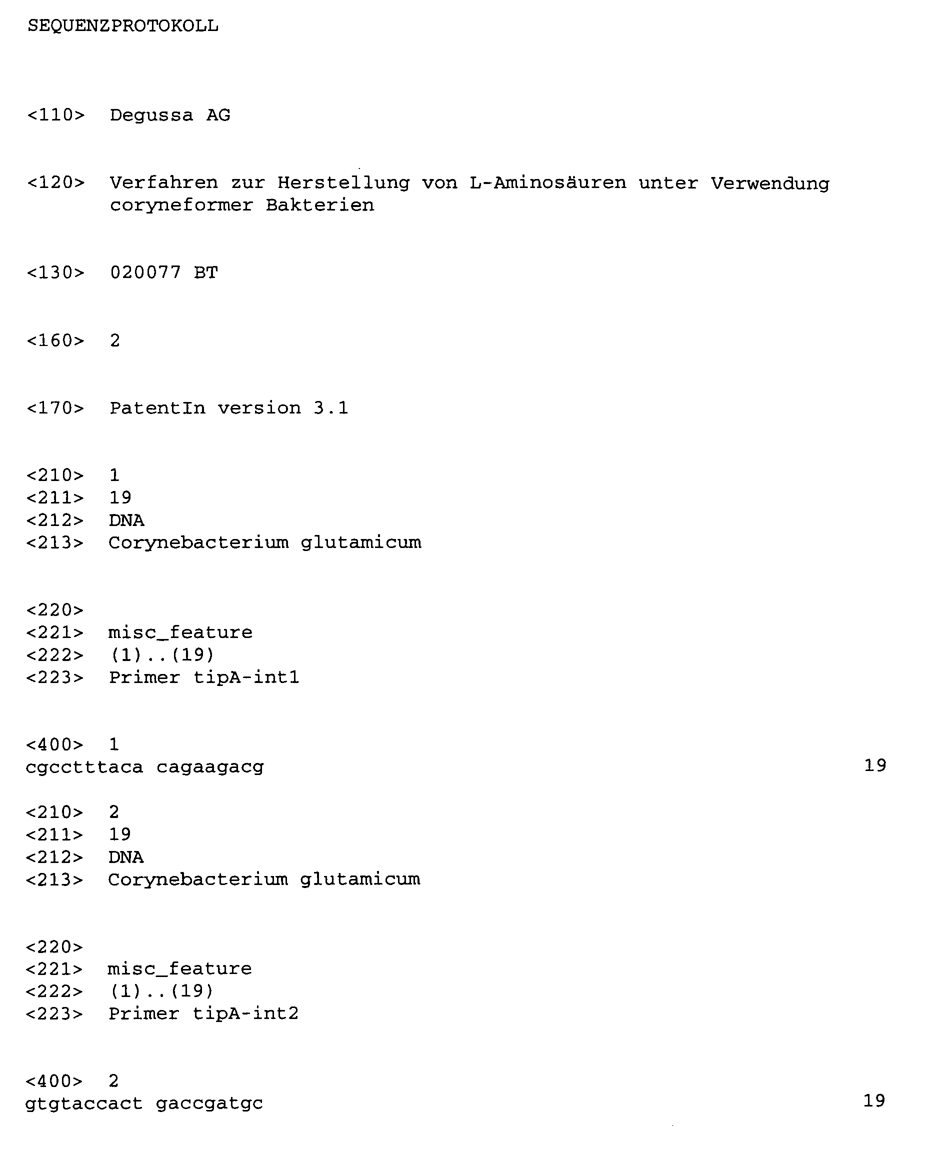

## Patentansprüche

1. Verfahren zur Herstellung von L-Aminosäuren durch Fermentation coryneformer Bakterien, **dadurch gekennzeichnet, dass** man Bakterien einsetzt, in denen man die für den Transkriptionsregulator TipA kodierende Nukleotidsequenz (tipA) abschwächt, insbesondere ausschaltet oder auf niedrigem Niveau exprimiert.

2. Verfahren gemäß Anspruch 1, **dadurch gekennzeichnet dass** man L-Lysin herstellt.

3. Verfahren zur Herstellung von L-Aminosäuren, insbesondere L-Lysin, **dadurch gekennzeichnet, dass** man folgende Schritte durchführt,
a) Fermentation der die gewünschte L-Aminosäure produzierenden coryneformen Bakterien, in denen man zumindest das für den Transkriptionsregulator TipA kodierende Gen abschwächt,
b) Anreicherung des gewünschten Produkts im Medium oder in den Zellen der Bakterien, und
c) Isolierung der gewünschten L-Aminosäure, wobei gegebenenfalls Bestandteile der Fermentationsbrühe und/oder Biomasse in Anteilen oder in ihren Gesamtmengen im Endprodukt verbleiben.

4. Verfahren gemäß Anspruch 1 oder 3, **dadurch gekennzeichnet, dass** man Bakterien einsetzt, in denen man zusätzlich weitere Gene des Biosyntheseweges der gewünschten L-Aminosäure verstärkt.

5. Verfahren gemäß Anspruch 1 oder 3, **dadurch gekennzeichnet, dass** man Bakterien einsetzt, in denen die Stoffwechselwege zumindest teilweise ausgeschaltet sind, die die Bildung der gewünschten L-Aminosäure verringern.

6. Verfahren gemäß Anspruch 1 oder 3, **dadurch gekennzeichnet, dass** man die Expression des Polynukleotids, das für den Transkriptionsregulator TipA kodiert, verringert.

7. Verfahren gemäß Anspruch 1 oder 3, **dadurch gekennzeichnet, daß** man die regulatorischen Eigenschaften des Polypeptids (Enzymproteins) verringert, für das die Transkriptionsregulator TipA Nukleotidsequenz (tipA) kodiert.

8. Verfahren gemäß Anspruch 1 oder 3, **dadurch gekennzeichnet, dass** man für die Herstellung von L-Lysin coryneforme Mikroorganismen fermentiert, in denen man gleichzeitig eines oder mehrere der Gene, ausgewählt aus der Gruppe
8.1 das für eine feed-back resistente Aspartatkinase kodierende Gen lysC,
8.2 das für den Lysin-Export kodierende Gen lysE,
8.3 das für die Glyceraldehyd-3-Phosphat Dehydrogenase kodierende Gen gap,
8.4 das für die Pyruvat Carboxylase kodierende Gen pyc,
8.5 das für die Glucose-6-Phosphat Dehydrogenase kodierende Gen zwf,
8.6 das für die Malat:Chinon Oxidoreduktase kodierende Gen mqo,
8.7 das für das Zwa1-Protein kodierende Gen zwa1,
8.8 das für die Triosephosphat Isomerase kodierende Gen tpi,
8.9 das für die 3-Phosphoglycerat Kinase kodierende Gen pgk,
8.10 das für die Dihydrodipicolinat-Synthase kodierende Gen dapA,
verstärkt insbesondere überexprimiert.

9. Verfahren gemäß Anspruch 1 oder 3, **dadurch gekennzeichnet, dass** man zur Herstellung von L-Aminosäuren coryneforme Mikroorganismen fermentiert, in denen man gleichzeitig eines oder mehrere der Gene, ausgewählt aus der Gruppe
9.1 das für ein Katabolit-Kontroll-Protein A kodierende Gen ccpA1,
9.2 das für die Phosphoenolpyruvat-Carboxykinase kodierende pck-Gen,
9.3 das für die Glucose-6-Phosphat Isomerase kodierende pgi-Gen,
9.4 das für die Pyruvat-Oxidase kodierende Gen poxB,
9.5 das für die Fruktose-Bisphosphat Aldolase kodierende Gen fda, oder
9.6 das für das Zwa2-Protein kodierende Gen zwa2,
abschwächt.

10. Verfahren gemäß einem oder mehreren der Ansprüche 1-9, **dadurch gekennzeichnet, daß** man Mikroorganismen der Art Corynebacterium glutamicum einsetzt.

11. Coryneforme Bakterien, in denen zumindest das für den Transkriptionsregulator TipA kodierende Gen abgeschwächt vorliegt.
